# EUROPÄISCHE PATENTSCHRIFT

(11) **EP 4 359 318 B1**
(45) Veröffentlichungstag und Bekanntmachung des Hinweises auf die Patenterteilung: **27.11.2024**
(21) Anmeldenummer: 23725994.0
(22) Anmeldetag: 09.05.2023
(51) Int. Cl.: B65D 75/08, B65D 65/12, B65D 81/07

(54) **UMVERPACKUNG FÜR EIN MEDIZINISCHES PRODUKT UND VERFAHREN ZUR UMVERPACKUNG EINES MEDIZINISCHES PRODUKTS**
OUTER PACKAGING FOR A MEDICAL PRODUCT AND METHOD FOR PACKAGING A MEDICAL PRODUCT
EMBALLAGE SECONDAIRE POUR UN PRODUIT MÉDICAL ET PROCÉDÉ D'EMBALLAGE D'UN PRODUIT MÉDICAL

(30) Priorität: 09.05.2022 DE 102022111498
(43) Veröffentlichungstag der Anmeldung: 01.05.2024
(73) Patentinhaber: Aesculap AG, 78532 Tuttlingen (DE)
(72) Erfinder: ETZEL, Florian, 71364 Winnenden (DE); WEBER, Stefanie, 78050 Villingen-Schwenningen (DE)
(74) Vertreter: Winter, Brandl - Partnerschaft mbB
(86) Internationale Anmeldenummer: PCT/EP2023/062214
(87) Internationale Veröffentlichungsnummer: WO 2023/217748

(56) Entgegenhaltungen:
- EP-B1- 2 739 238
- EP-B1- 3 357 444
- WO-A1-2021/212020
- DE-U1- 9 317 306
- FR-A- 893 332
- JP-U- S4 728 476
- US-A1- 2011 024 324

## Beschreibung

Die Offenbarung betrifft eine faltbare Umverpackung für ein medizinisches Produkt, sowie ein Verfahren Umverpackung eines medizinischen Produkt unter Verwendung der faltbaren Umverpackung.

### Hintergrund der Offenbarung

Medizinprodukte, wie beispielsweise Knochenimplantate, Knochenplatten, intramedulläre Vorrichtungen, künstliche Gelenke oder Ähnliches, werden üblicherweise in einer kontrollierten Umgebung, insbesondere in einem Reinraum, verpackt und dann vor einem Transport an einen Kunden, wie z. B. ein Krankenhaus, sterilisiert. In der Regel wird hierfür ein doppeltes Sterilbarrieresystem verwendet. Die doppelte Sterilbarriere kann dabei aus zwei Kunststoffbeuteln (Primärverpackungen) bestehen. Das Medizinprodukt wird in einen ersten, inneren Kunststoffbeutel eingeführt, der vakuumiert und versiegelt wird. Noch in der kontrollierten Umgebung wird der versiegelte erste Kunststoffbeutel dann in einen zweiten, äußeren Kunststoffbeutel eingelegt, der wiederum ebenfalls vakuumiert und versiegelt wird. Es können auch mehr als zwei Kunststoffbeutel oder nur ein einziger Kunststoffbeutel als Sterilbarriere verwendet werden. Der oder die Kunststoffbeutel mit dem Medizinprodukt werden dann für den Versand und Transport in eine Umverpackungsschachtel (Sekundärverpackung), einen Karton oder ähnliches, eingelegt und anschließend transportiert.

Die Sterilbarriere muss dabei intakt bleiben, um eine Kontamination des Medizinprodukts zu vermeiden. Der Kunststoffbeutel ist jedoch form instabil. Wenn die Kunststoffbeutel während des Transports jedoch lose in einer herkömmlichen (genormten) Umverpackungsschachtel angeordnet werden, können hierbei durch aneinander reibende oder schlagende Kunststoffbeutel und Medizinprodukte Beschädigungen an den Kunststoffbeuteln auftreten, was die Gewährleistung der Sterilität beeinträchtigt. Insbesondere große und schwere Medizinprodukte können die Kunststoffbeutel beschädigen, wenn sie sich in der Sekundärverpackung bewegen und gegen die Innenwände der Sekundärverpackung stoßen. Daher sind üblicherweise zusätzliche Schutzkomponenten, wie z. B. Schaumstoffe oder andere Polstermaterialien vorgesehen, welche zu den Gesamtkosten und der Komplexität des Umverpackungssystems nachteilig beitragen.

Um Beschädigungen zu vermeiden, werden die Kunststoffbeutel alternativ in faltbare Einzelverpackungen (Sekundärverpackungen) verpackt, welche wiederum in der Umverpackungsschachtel (Tertiärverpackung) angeordnet werden. So zeigt beispielsweise EP 2 739 238 B1 ein Umverpackungssystem für eine medizinische Vorrichtung bzw. ein Medizinprodukt mit einem versiegelten Kunststoffbeutel als sterile Primärverpackung für das Medizinprodukt und einer faltbaren Sekundärverpackung, welche einen Innenraum zur Aufnahme des Kunststoffbeutels definiert.

Darüber hinaus ist in EP 3 357 444 B1 eine Umverpackungsanordnung für eine medizinische Vorrichtung offenbart, welche wenigstens zwei Kunststoffbeutel aufweist. Die Umverpackungsanordnung hat ferner ein Tragelement, welches in einer zusammengebauten Konfiguration gegen eine Bewegung relativ zu dem inneren der zumindest zwei Kunststoffbeutel gesichert ist. Diese Sicherung erfolgt dabei durch die Kunststoffbeutel selbst, welche um das Tragelement gefaltet sind. Darüber hinaus sind auch in DE 100 14 419 A1, EP 3 789 314 A1, US 2010 / 0 320 112 A1, DE 698 13 860 T2, DE 10 2005 038 491 B3, DE 93 17 306 U1 oder FR 893 332 A faltbare Umverpackungen gezeigt.

Jedoch sind die bekannten Umverpackungen immer von den Abmessungen des Medizinprodukts abhängig und darüber hinaus nicht flexibel in Umverpackungsschachteln unterschiedlicher Größen einsetzbar. Auch unter dem Aspekt der Nachhaltigkeit bzw. möglichst einfachen Trennung der Umverpackungsmaterialien bei der Entsorgung sind die bisherigen Lösungen nachteilig, da sie aus verschiedenen Materialien/Verbunde (Kunststoff/Papier) bestehen.

### Zusammenfassung der Offenbarung

Es sind die Aufgaben und Ziele der Offenbarung, die Nachteile aus dem Stand der Technik zu beheben oder wenigstens zu mindern und insbesondere soll eine faltbare Umverpackung bereitgestellt werden, welche bei einfacher Entsorgung einen sicheren Transport steril verpackter Medizinprodukte in genormten Umverpackungsschachteln (Tertiärverpackung) ermöglicht.

Die Aufgaben und Ziele werden hinsichtlich einer gattungsgemäßen Umverpackung erfindungsgemäß durch den Gegenstand des Anspruchs 1 gelöst.

Die (beispielsweise aus Kartonagenmaterial gefertigte) Umverpackung beinhaltet demgemäß demzufolge eine Basisplatte, die mittels zweier parallel beabstandeter Klappfalzlinien in drei Segmente unterteilt ist, eine Schwenkplatte, die über eine senkrecht zu den Klappfalzlinien ausgerichtete Schwenkfalzlinie mit der Basisplatte gekoppelt ist, mittels der in die Schwenkplatte sich geradlinig verlängernden Klappfalzlinien ebenfalls in drei Segmente unterteilt ist, und einen fensterartigen Ausschnitt oder Durchbruch im mittleren der drei Segmente aufweist, und eine Seitenplatte, die über eine parallel zu der Schwenkfalzlinie ausgerichtete Umlegfalzlinie mit der Schwenkplatte gekoppelt ist, und mittels der in die Seitenplatte sich geradlinig weiter verlängernden Klappfalzlinien ebenfalls in drei Segmente unterteilt ist.

Erfindungsgemäß erstreckt sich die Schwenkfalzlinie dabei über die gesamte Länge der Basisplatte und der Schwenkplatte.

Erfindungsgemäß weist das mittlere der drei Segmente der Basisplatte ein Schnittmuster in Form einer Vielzahl an Längsschnitten auf. Vorteilhafte Ausführungsformen sind in den Unteransprüchen beansprucht und werden nachfolgend erläutert.

Ferner kann es von Vorteil sein, wenn sich die Umlegfalzlinie über die gesamte Länge der Seitenplatte und der Schwenkplatte erstreckt.

Gemäß einer vorteilhaften Ausgestaltung kann die Umverpackung aus einem herkömmlichen Verpackungsmaterial, wie z.B. Karton oder Pappe, hergestellt sein.

Darüber hinaus betrifft die Erfindung ein Verfahren zur Umverpackung eines medizinischen Produkts unter Verwendung einer faltbaren Umverpackung gemäß Anspruch 1 oder 2 mit den folgenden Verfahrensschritten:
- Auflegen eines medizinischen Produkts auf das mittlere der drei Segmente der Basisplatte;
- Schwenken der Schwenkplatte längs der Schwenkfalzlinie bis die Schwenkplatte unter Hindurchragen des medizinischen Produkts durch den fensterartigen Ausschnitt auf der Basisplatte aufliegt;
- Umlegen der Seitenplatte längs der Umlegfalzlinie, um die Basisplatte sandwichartig zwischen der Seitenplatte und der Schwenkplatte aufzunehmen und
- Umklappen der jeweils beiden äußeren der drei Segmente der Basisplatte, Schwenkplatte und Seitenplatte längs der jeweiligen Klappfalzlinien in Richtung hin zum medizinischen Produkt.

Bevorzugt kann das medizinische Produkt vor dem Auflegen auf das mittlere der drei Segmente der Basisplatte in einer sterilen Umverpackung verpackt werden. Dabei kann das Medizinprodukt in einem Kunststoffbeutel als sterile Umverpackung / sterile Barriere / Sterilbarrieresystem / Primärverpackung verpackt werden. Alternativ ist es auch denkbar, dass das Medizinprodukt in zwei oder mehreren Kunststoffbeuteln, beispielsweise in Form eines Doppelbarrieresystems, verpackt wird.

Gemäß einer vorteilhaften Weiterbildung kann die Umverpackung mitsamt dem medizinischen Produkt nach dem Umklappen der jeweils beiden äußeren der drei Segmente der Basisplatte, Schwenkplatte und Seitenplatte längs der jeweiligen Klappfalzlinien in Richtung hin zum medizinischen Produkt in einer, vorzugsweise genormten, Umverpackungsschachtel verpackt werden. Von besonderem Vorteil ist es dabei, wenn die Umverpackungsschachtel aus demselben Verpackungsmaterial wie die faltbare Umverpackung, beispielsweise Pappe oder Karton, hergestellt ist. Selbstverständlich sind auch andere Verpackungsmaterialien denkbar, solange die Umverpackung und die Umverpackungsschachtel aus demselben Material hergestellt sind.

Offenbarungsgemäß kann es zudem denkbar sein, dass der fensterartige Ausschnitt an Abmessungen des Medizinprodukts angepasst bzw. anpassbar (z.B. durch Ausschneiden) ist. Wenn das Medizinprodukt beispielsweise einen rechteckigen Querschnitt oder einen ellipsenförmigen Querschnitt hat, kann es zweckmäßig sein, wenn der Ausschnitt ebenfalls rechteckig bzw. ellipsenförmig ausgebildet ist.

In anderen Worten ausgedrückt betrifft die Offenbarung einem Zuschnitt der Umverpackung mit der Basisplatte, der Schwenkplatte und der Seitenplatte, welche in dieser Reihenfolge nebeneinander angeordnet und über die Schwenkfalzlinie und die Umlegfalzlinie verbunden sind, und Falten des Zuschnitts entlang der Schwenkfalzlinie und der Umlegfalzlinie ein auf der Basisplatte aufgelegtes Medizinprodukt relativ zu der Umverpackung fixiert. Bevorzugt kann der Zuschnitt ferner eine erste Seitenlasche und eine zweite Seitenlasche aufweisen, welche über die erste Klappfalzlinie und die zweite Klappfalzlinie mit der Schwenkplatte und der Basisplatte verbunden sind, so dass sich beim Falten der Seitenlaschen entlang der ersten Klappfalzlinie und der vierten Klappfalzlinie zwischen den Seitenlaschen ein Hohlraum ausbildet, in welchen das Medizinprodukt vorragt. Vorzugsweise können die Schwenkfalzlinie und die Umlegfalzlinie dabei parallel zueinander angeordnet sein, und die erste Klappfalzlinie und die zweite Klappfalzlinie können jeweils senkrecht auf der Schwenkfalzlinie und der Umlegfalzlinie angeordnet sein. Besonders bevorzugt können die Seitenlaschen und die Basisplatte die Umverpackung in einer, vorzugsweise genormten, Umverpackungsschachtel (Tertiärverpackung) ortsfest festlegen.

Mit anderen Worten betrifft die Offenbarung weiter ein Verfahren zum Verpacken eines Medizinprodukts in einer, vorzugsweise erfindungsgemäßen, faltbaren Umverpackung mit den folgenden Schritten:
- Auflegen des Medizinprodukts auf die Basisplatte der Umverpackung;
- Falten der Umverpackung entlang einer Schwenkfalzlinie und einer Umlegfalzlinie, um das Medizinprodukt relativ zu der Umverpackung zu fixieren; und
- Falten der Umverpackung entlang einer ersten Klappfalzlinie und einer zweiten Klappfalzlinie, um einen Hohlraum auszubilden, in welchen das Medizinprodukt vorragt.

### Kurzbeschreibung der Figuren

Die Offenbarung wird nachfolgend anhand einer bevorzugten Ausführungsform mit Hilfe von Figuren näher erläutert. Es zeigen:
- Fig. 1: einen Zuschnitt einer Umverpackung;
- Fig. 2: einen ersten Schritt zum Verpacken eines Medizinprodukts mit der Umverpackung;
- Fign. 3, 4: einen zweiten Schritt zum Verpacken des Medizinprodukts mit der Umverpackung;
- Fign. 5, 6: einen dritten Schritt zum Verpacken des Medizinprodukts mit der Umverpackung;
- Fig. 7: einen vierten Schritt zum Verpacken des Medizinprodukts mit der Umverpackung;
- Fig. 8: einen Schritt zum Verpacken des mit der Umverpackung verpackten Medizinprodukts in einer Tertiärverpackung;
- Fig. 9: das in der Tertiärverpackung verpackte Medizinprodukt;
- Fign. 10, 11: in der Tertiärverpackung verpackte Medizinprodukte;
- Fig. 12: einen Zuschnitt einer Umverpackung gemäß einer bevorzugten Ausführungsform der Erfindung;
- Fig. 13: ein in der Umverpackung gemäß der bevorzugten Ausführungsform der Erfindung verpacktes Medizinprodukt; und
- Fig. 14: das in der Umverpackung gemäß der bevorzugten Ausführungsform der Erfindung verpackte Medizinprodukt in der Tertiärverpackung

Die Figuren sind schematischer Natur und dienen lediglich dem Verständnis der Erfindung. Gleiche Elemente sind mit denselben Bezugszeichen versehen.

### Detaillierte Beschreibung einer bevorzugten Ausführungsform

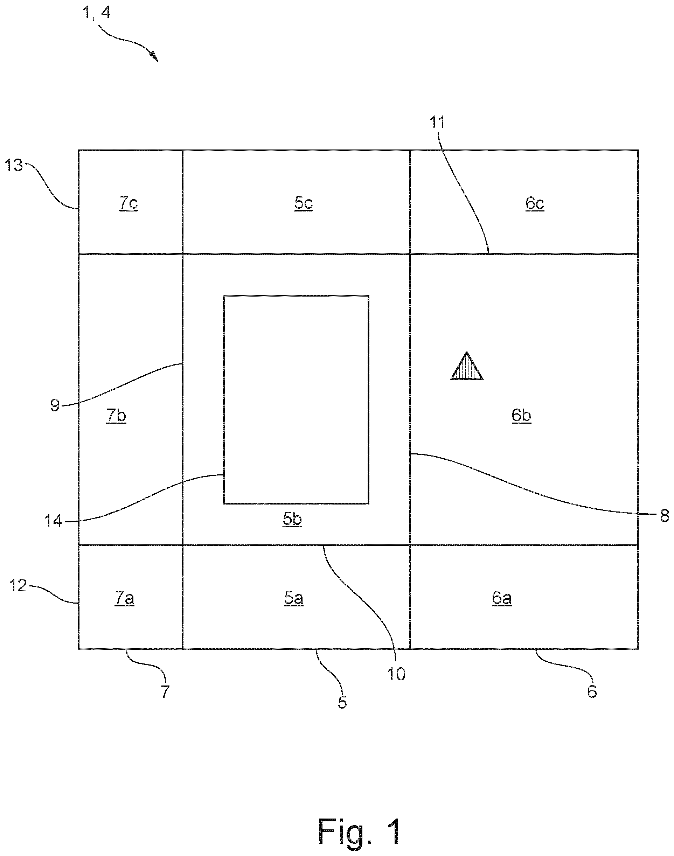

Fig. 1 zeigt eine faltbare (Sekundär-) Umverpackung 1 für ein in einer sterilen Primärverpackung / sterile Barriere 2 verpacktes Medizinprodukt 3 in einem aufgefalteten Zustand. Mit anderen Worten zeigt Fig. 1 einen Zuschnitt oder Faltbogen 4 der Umverpackung 1 gemäß einer bevorzugten Ausführungsform.

Die Umverpackung 1 weist eine Schwenkplatte 5, eine Basisplatte 6 und eine Seitenplatte 7 auf, welche jeweils über eine Schwenkfalzlinie 8 und eine Umlegfalzlinie 9 miteinander verbunden sind. Dabei ist die Schwenkplatte 5 über eine Schwenkfalzlinie 8 mit der Basisplatte 6 verbunden und über eine Umlegfalzlinie 9 mit der Seitenplatte 7 verbunden. In der in Fig. 1 gezeigten Draufsicht sind folglich die Seitenplatte 7, die Schwenkplatte 5 und die Basisplatte 6 nebeneinander angeordnet und jeweils über die Umlegfalzlinie 9 bzw. die Schwenkfalzlinie 8 miteinander verbunden. Die Schwenkfalzlinie 8 und die Umlegfalzlinie 9 sind im Wesentlichen parallel angeordnet und unterteilen den Zuschnitt 4 (in Fig. 1 vertikal) in drei Elemente, nämlich die Seitenplatte 7, die Schwenkplatte 5 und die Basisplatte 6. Dabei ist eine Fläche der Schwenkplatte 5 in einer Draufsicht, wie in Fig. 1 gezeigt, gleich einer Fläche der Basisplatte 6, so dass, wie nachstehend näher beschrieben, die Schwenkplatte 5, wenn sie entlang der Schwenkfalzlinie 8 über die Basisplatte 6 gefaltet wird, diese vollständig überdeckt.

Wie in Fig. 1 gezeigt, weist die Umverpackung 1 bzw. der Zuschnitt 4 ferner eine erste Klappfalzlinie 10 und eine zweite Klappfalzlinie 11 auf. Die erste Klappfalzlinie 10 und die zweite Klappfalzlinie 11 sind dabei im Wesentlichen senkrecht zu der Schwenkfalzlinie 8 und der Umlegfalzlinie 9 angeordnet. Der Zuschnitt 4 wird somit in seiner Erstreckungsrichtung entlang der Schwenkfalzlinie 8 und der Umlegfalzlinie 9 in drei (horizontale) Segmente unterteilt. Anders ausgedrückt werden die Schwenkplatte 5, die Basisplatte 6 und die Seitenplatte 7 durch die erste Klappfalzlinie 10 und die zweite Klappfalzlinie 11 jeweils in drei übereinander angeordnete Segmente unterteilt. So wird die Schwenkplatte 5 in ein erstes, unteres Schwenkplattensegment 5a, ein zweites, mittleres Schwenkplattensegment 5b und eine drittes, oberes Schwenkplattensegment 5c unterteilt. Ebenso wird die Basisplatte 6 durch die erste Klappfalzlinie 10 und die zweite Klappfalzlinie 11 in ein erstes, unteres Basisplattensegment 6a, ein zweites, mittleres Basisplattensegment 6b und ein drittes, oberes Basisplattensegment 6c unterteilt. Die Seitenplatte 7 wird durch die erste Klappfalzlinie 10 und die zweite Klappfalzlinie 11 in ein erstes, unteres Seitenplattensegment 7a, ein zweites, mittleres Seitenplattensegment 7b und ein drittes, oberes Seitenplattensegment 7c unterteilt.

Dabei sind die ersten und dritten Segmente, d.h. das erste Schwenkplattensegment 5a und das dritte Schwenkplattensegment 5c, das erste Basisplattensegment 6a und das dritte Basisplattensegment 6c sowie das erste Seitenplattensegment 7a und das dritte Seitenplattensegment 7c, jeweils gleich groß bzw. haben dieselben Außenabmessungen. D.h. in der Draufsicht in Fig. 1 ist der horizontale Abstand zwischen der unteren, horizontalen Außenkante des Zuschnitts 4 und der ersten Klappfalzlinie 10 gleich dem horizontalen Abstand zwischen der oberen, horizontalen Außenkante des Zuschnitts 4 und der zweiten Klappfalzlinie 11.

Wie in Fig. 1 zu erkennen, wird der Zuschnitt 4 der Umverpackung 1 durch die Falzlinien 8, 9, 10, 11 in die neun Segmente 5a, 5b, 5c, 6a, 6b, 6c, 7a, 7b, 7c unterteilt. Dabei ist die Schwenkfalzlinie 8 zwischen den Segmenten 5a, 5b, 5c, welche die Schwenkplatte 5 ausbilden, und den Segmenten 6a, 6b, 6c, welche die Basisplatte 6 ausbilden, angeordnet. Die Umlegfalzlinie 9 ist zwischen den Segmenten 5a, 5b, 5c der Schwenkplatte 5 und den Segmenten 7a, 7b, 7c der Seitenplatte 7 ausgebildet. Die erste Klappfalzlinie 10 ist zwischen den Segmenten 5a, 6a, 7a, welche eine erste Seitenlasche 12 ausbilden, und den Segmenten 5b, 6b, 7b ausgebildet, wohingegen die zweite Klappfalzlinie 11 zwischen den Segmenten 5b, 6b, 7b und den Segmenten 5c, 6c, 7c einer zweiten Seitenlasche 13 angeordnet sind.

In der Schwenkplatte 5, insbesondere in dem zweiten, mittleren Schwenkplattensegment 5b, ist, wie in Fig. 1 gezeigt, ein Ausschnitt 14 ausgebildet. In der bevorzugten Ausführungsform ist der Ausschnitt 14 in Form einer rechteckigen Durchgangsöffnung ausgeformt. Die Form und die Abmessungen des Ausschnitts 14 sind dabei, wie nachstehend näher beschrieben, an das mit der Umverpackung 1 zu verpackende Medizinprodukt 3 angepasst. So kann der Ausschnitt 14 beispielsweise auch kreis- oder ellipsenförmig oder in Form eines beliebigen Vielecks ausgeformt sein.

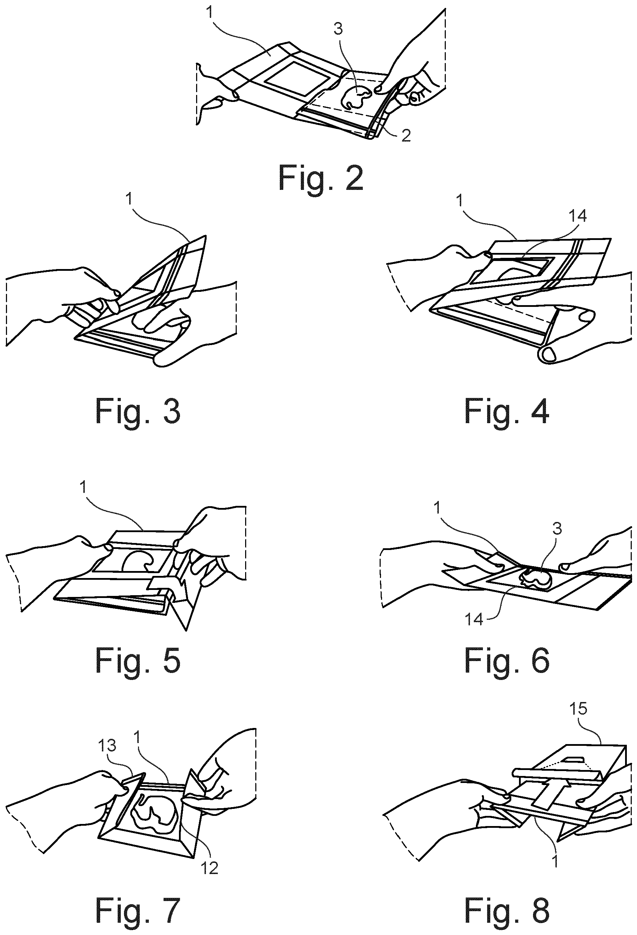

Um das Medizinprodukt 3 mit der Umverpackung 1 zu verpacken, wird zunächst in einem ersten, in Fig. 2 gezeigten Schritt das in der Primärverpackung 2 sterilverpackte Medizinprodukt 3 auf die Basisplatte 6, insbesondere das zweite, mittlere Basisplattensegment 6b, gelegt. Die Primärverpackung 2 kann dabei, wie in Fig. 2 gezeigt, über das zweite Basisplattensegment 6b hinausragen, solange das Medizinprodukt 3 in der Primärverpackung 2 auf dem zweiten Basisplattensegment 6b angeordnet ist. Anders ausgedrückt, dient die Basisplatte 6 als Aufnahme für das in der Primärverpackung 2 verpackte Medizinprodukt 3.

In einem zweiten, in den Fign. 3 und 4 gezeigten Schritt wird die Schwenkplatte 5 mitsamt der Seitenplatte 7 entlang der Schwenkfalzlinie 8 hin zu der Basisplatte 6 gefaltet, so dass die Schwenkplatte 5 die Basisplatte 6 im Wesentlichen überdeckt. Das Medizinprodukt 3 in der Primärverpackung 2 ragt durch den Ausschnitt 14 der Schwenkplatte 5 vor. D.h. der Ausschnitt 14 umgreift/umschließt das Medizinprodukt 3 in dem zusammengefalteten Zustand, um es so relativ zu der Umverpackung 1 zu fixieren.

Anschließend wird die Seitenplatte 7 in einem dritten, in den Fign. 5 und 6 gezeigten Schritt entlang der Umlegfalzlinie 9 nach innen hin zu einer Unterseite der Basisplatte 6 gefaltet bzw. umgeklappt, um so die Schwenkplatte 5 auf der Basisplatte 6 zu halten. Anders ausgedrückt wird die Seitenplatte 7 entlang der Umlegfalzlinie 9 gefaltet, um die Basisplatte 6 zwischen sich und der Schwenkplatte 5 sandwichartig zu halten.

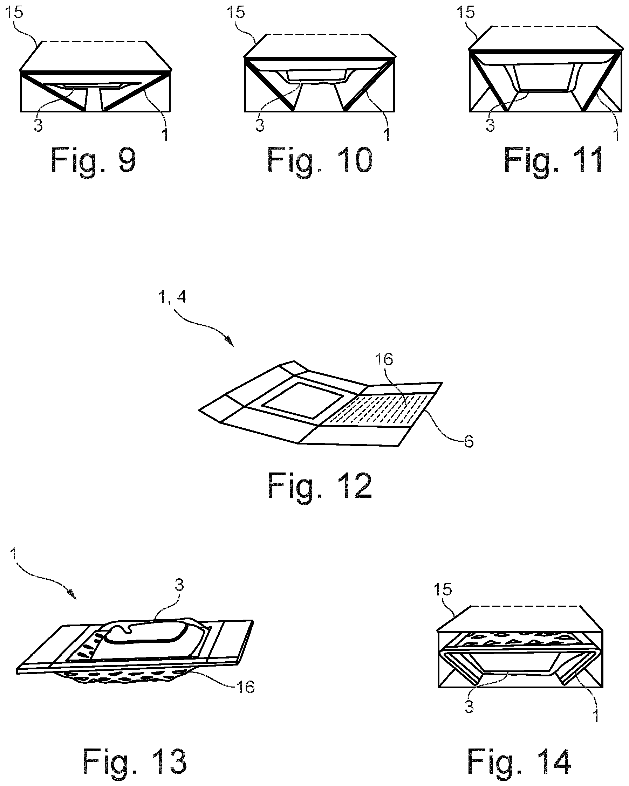

In einem vierten, in Fig. 7 gezeigten Schritt werden die Seitenlaschen 12 und 13, d.h. die übereinanderliegenden Segmente 5a, 6a, 7a sowie die übereinanderliegenden Segmente 5b, 6b, 7b, nach oben in Richtung hin zu dem durch den Ausschnitt 14 vorragenden Medizinprodukt 3 gefaltet. Wenn nun das in der Umverpackung 1 verpackte Medizinprodukt 3 beispielsweise für den Transport oder Versand in eine, vorzugsweise genormte, Umverpackungsschachtel (Tertiärverpackung) 15 eingelegt wird, klappen die Seitenlaschen 12, 13 aufgrund der Eigenelastizität des Umverpackungsmaterials wieder nach außen und stoßen dabei gegen Innenflächen der Umverpackungsschachtel 15. D.h. die Umverpackung 1 gemäß der bevorzugten Ausführungsform liegt, wie in Fig. 9 gezeigt, in dem zusammengefalteten Zustand mit der Unterseite der Basisplatte 6, d.h. der dem Medizinprodukt 3 abgewandten Seite, an einer ersten Innenfläche der Umverpackungsschachtel 15 an. Die Kanten der Seitenlaschen 12, 13 der Umverpackung liegen dabei auf einer zweiten Innenfläche der Umverpackungsschachtel 15 gegenüber der ersten Innenfläche an, so dass die Umverpackung 1 in der Umverpackungsschachtel 15 gehalten wird. Mit anderen Worten ausgedrückt, verspreizt sich die Umverpackung 1 in der Umverpackungsschachtel 15.

Wie in Fig. 9 gezeigt, bilden die Seitenlaschen 12, 13 dabei zwischen sich einen Hohlraum aus, in welchen das Medizinprodukt 3 vorragen kann. D.h. das Medizinprodukt 3 wird durch die Umverpackung 1 in der Umverpackungsschachtel 15 fixiert ohne an einer der Innenflächen der Umverpackungsschachtel 15 anzuliegen bzw. mit der Umverpackungsschachtel 15 in Kontakt zu geraten. So kann eine Beschädigung der Primärverpackung 2 und eine damit verbundene Beeinträchtigung der Sterilität des Medizinprodukts 3 verhindert werden.

Die Umverpackung 1 kann dabei auch, wie in den Fign. 10, 11 gezeigt, für unterschiedlich große Medizinprodukte 3 verwendet werden. Wenn ein großes Medizinprodukt 3 in der Umverpackung 1 bzw. in der Umverpackungsschachtel 15 verpackt wird, stehen die Seitenlaschen 12, 13 im Vergleich zu kleineren Medizinprodukten 3 senkrechter und bilden somit einen größeren Hohlraum zwischen sich aus.

In den Fign. 12 bis 14, ist eine Umverpackung 1 gemäß einer bevorzugten Ausführungsform der Erfindung abgebildet. Dabei weist das zweite, mittlere Basisplattensegment 6b der Basisplatte 6 ein Schnittmuster 16 auf. Das Schnittmuster 16 ist gemäß der bevorzugten Ausführungsform der Erfindung in Form einer Vielzahl von einzelnen (zeilenförmig angeordneten) Längsschnitten in der Basisplatte 6 ausgeführt. Wenn die Umverpackung 1 gemäß der bevorzugten Ausführungsform der Erfindung zusammengefaltet und in die Umverpackungsschachtel 15 eingelegt wird, sorgt das Schnittmuster 16 für eine zusätzliche Dämpfung zwischen der Umverpackungsschachtel 15 und dem Medizinprodukt 3. Zudem erhält die Basisplatte 6, insbesondere deren mittleres Basisplattensegment 6b aufgrund der darin angebrachten Längsschnitte (Längsschlitze) eine gegenüber einer ungeschnittenen Platte höhere Flexibilität, sodass sich diese an die äußere Form eines darauf aufgelegten Produkts näherungsweise anpassen kann (siehe insbesondere Fig. 13).

In der bevorzugten Ausführungsform der Erfindung ist die Umverpackung 1 aus einem herkömmlichen Umverpackungsmaterial, wie beispielsweise Pappe oder Karton. Da auch die Umverpackungsschachtel 15 aus dem herkömmlichen Umverpackungsmaterial hergestellt ist, ist ein Trennen der Umverpackungskomponenten bei der Entsorgung nicht mehr notwendig. Selbstverständlich kann auch ein anderes Material für die Umverpackung 1 und die Umverpackungsschachtel 15 verwendet werden, solange für die Umverpackungskomponenten dasselbe Material verwendet wird.

### Bezugszeichenliste

- 1: Umverpackung
- 2: Primärverpackung
- 3: Medizinprodukt
- 4: Zuschnitt
- 5: Schwenkplatte
- 5a: erstes Schwenkplattensegment
- 5b: zweites Schwenkplattensegment
- 5c: drittes Schwenkplattensegment
- 6: Basisplatte
- 6a: erstes Basisplattensegment
- 6b: zweites Basisplattensegment
- 6c: drittes Basisplattensegment
- 7: Seitenplatte
- 7a: erstes Seitenplattensegment
- 7b: zweites Seitenplattensegment
- 7c: drittes Seitenplattensegment
- 8: Schwenkfalzlinie
- 9: Umlegfalzlinie
- 10: erste Klappfalzlinie
- 11: zweite Klappfalzlinie
- 12: erste Seitenlasche
- 13: zweite Seitenlasche
- 14: Ausschnitt
- 15: Umverpackungsschachtel
- 16: Schnittmuster

## Patentansprüche

1. Faltbare Umverpackung (1) für ein medizinisches Produkt (3) mit:
- einer Basisplatte (6), die mittels zweier parallel beabstandeter Klappfalzlinien (10, 11) in drei Segmente (6a, 6b, 6c) unterteilt ist,
- einer Schwenkplatte (5), die
- über eine senkrecht zu den Klappfalzlinien (10, 11) ausgerichtete Schwenkfalzlinie (8) mit der Basisplatte (6) gekoppelt ist,
- mittels der in die Schwenkplatte (5) sich geradlinig verlängernden Klappfalzlinien (10, 11) ebenfalls in drei Segmente (5a, 5b. 5c) unterteilt ist, und
- einen fensterartigen Ausschnitt (14) (oder Durchbruch) im mittleren (5b) der drei Segmente (5a, 5b ,5c) aufweist, und
- einer Seitenplatte (7), die
- über eine parallel zu der Schwenkfalzlinie (8) ausgerichtete Umlegfalzlinie (9) mit der Schwenkplatte (5) gekoppelt ist, und
- mittels der in die Seitenplatte (7) sich geradlinig weiter verlängernden Klappfalzlinien (10, 11) ebenfalls in drei Segmente (7a, 7b, 7c) unterteilt ist,
wobei sich die Schwenkfalzlinie (8) über die gesamte Länge der Basisplatte (6) und der Schwenkplatte (5) erstreckt,
**dadurch gekennzeichnet, dass** das mittlere (6b) der drei Segmente (6a, 6b, 6c) der Basisplatte (6) ein Schnittmuster (16) in Form einer Vielzahl an einzelnen Längsschnitten aufweist.

2. Umverpackung (1) nach Anspruch 1, **dadurch gekennzeichnet, dass** sich die Umlegfalzlinie (9) über die gesamte Länge der Seitenplatte (7) und der Schwenkplatte (5) erstreckt.

3. Verfahren zur Umverpackung eines medizinischen Produkts (3) unter Verwendung einer faltbaren Umverpackung (1) gemäß Anspruch 1 oder 2 mit den folgenden Verfahrensschritten:
- Auflegen eines medizinischen Produkts (3) auf das mittlere (6b) der drei Segmente (6a, 6b, 6c) der Basisplatte (6);
- Schwenken der Schwenkplatte (5) längs der Schwenkfalzlinie (8) bis die Schwenkplatte (5) unter Hindurchragen des medizinischen Produkts (3) durch den fensterartigen Ausschnitt (14) auf der Basisplatte (6) aufliegt;
- Umlegen der Seitenplatte (7) längs der Umlegfalzlinie (9), um die Basisplatte (6) sandwichartig zwischen der Seitenplatte (7) und der Schwenkplatte (5) aufzunehmen und
- Umklappen der jeweils beiden äußeren (5a, 5c, 6a, 6c, 7a, 7c) der drei Segmente der Basisplatte (6), Schwenkplatte (5) und Seitenplatte (7) längs der jeweiligen Klappfalzlinien (10, 11) in Richtung hin zum medizinischen Produkt (3).

4. Verfahren nach Anspruch 3, **dadurch gekennzeichnet, dass** das medizinische Produkt (3) vor dem Auflegen auf das mittlere (6b) der drei Segmente (6a, 6b, 6c) der Basisplatte (6) in einer sterilen Umverpackung (1) verpackt wird.

5. Verfahren nach Anspruch 3 oder 4, **dadurch gekennzeichnet, dass** die Umverpackung (1) mitsamt dem medizinischen Produkt (3) nach dem Umklappen der jeweils beiden äußeren (5a, 5c, 6a, 6c, 7a, 7c) der drei Segmente der Basisplatte (6), Schwenkplatte (5) und Seitenplatte (7) längs der jeweiligen Klappfalzlinien (10, 11) in Richtung hin zum medizinischen Produkt (3) in einer, vorzugsweise genormten, Umverpackungsschachtel (15) verpackt wird.

## Claims

1. A foldable outer packaging (1) for a medical product (3) with:
- a base plate (6), which is divided into three segments (6a, 6b, 6c) via two parallel spaced folding fold lines (10, 11),
- a pivot plate (5), which
- is coupled to the base plate (6) via a pivoting fold line (8) aligned perpendicular to the folding fold lines (10, 11),
- is also subdivided into three segments (5a, 5b, 5c) via the folding fold lines (10, 11) extending in a straight line into the pivot plate (5), and
- has a window-like cut-out (14) (or aperture) in the middle (5b) of the three segments (5a, 5b, 5c), and
- a side plate (7), which
- is coupled to the pivot plate (5) via a turn-over fold line (9) aligned parallel to the pivoting fold line (8), and
- is also divided into three segments (7a, 7b, 7c) via the folding fold lines (10, 11) that continue to extend in a straight line into the side plate (7),
wherein the pivoting fold line (8) extends over the entire length of the base plate (6) and of the pivot plate (5),
**characterized in that** the middle one (6b) of the three segments (6a, 6b, 6c) of the base plate (6) has a cutting pattern (16) in the form of a plurality of individual longitudinal cuts.

2. The outer packaging (1) according to claim 1, **characterized in that** the turn-over fold line (9) extends over the entire length of the side plate (7) and the pivot plate (5).

3. A method of packaging a medical device (3) using a foldable outer packaging (1) according to claim 1 or 2, comprising the following method steps:
- placing a medical product (3) on the middle (6b) of the three segments (6a, 6b, 6c) of the base plate (6);
- pivoting of the pivot plate (5) along the pivoting fold line (8) until the pivot plate (5) rests on the base plate (6) with the medical product (3) protruding through the window-like cut-out (14);
- turning over the side plate (7) along the turn-over fold line (9) to sandwich the base plate (6) between the side plate (7) and the pivot plate (5), and
- folding over the two outer segments (5a, 5c, 6a, 6c, 7a, 7c) of the three segments of the base plate (6), pivot plate (5) and side plate (7) along the respective folding fold lines (10, 11) in the direction of the medical product (3).

4. The method according to claim 3, **characterized in that** the medical product (3) is packaged in a sterile outer packaging (1) before being placed on the middle one (6b) of the three segments (6a, 6b, 6c) of the base plate (6).

5. The method according to claim 3 or 4, **characterized in that** the outer packaging (1) together with the medical product (3) is packaged in a, preferably standardized, outer packaging box (15) after folding over the two outer (5a, 5c, 6a, 6c, 7a, 7c) segments of the base plate (6), pivot plate (5) and side plate (7) along the respective folding fold lines (10, 11) in the direction of the medical product (3).

## Revendications

1. Emballage secondaire pliable (1) pour un produit médical (3) avec :
- une plaque de base (6) qui est divisée en trois segments (6a, 6b, 6c) au moyen de deux lignes de pliage rabattables (10, 11) espacées parallèlement,
- d'une plaque pivotante (5) qui
- est couplée à la plaque de base (6) par l'intermédiaire d'une ligne de pliage pivotante (8) orientée perpendiculairement aux lignes de pliage rabattables (10, 11),
- est aussi divisée en trois segments (5a, 5b, 5c) au moyen des lignes de pliage rabattables (10, 11) se prolongeant en ligne droite dans la plaque pivotante (5), et
- une découpe (14) (ou perçage) en forme de fenêtre dans le segment central (5b) des trois segments (5a, 5b, 5c), et
- une plaque latérale (7) qui
- est couplée à la plaque pivotante (5) par l'intermédiaire d'une ligne de pliage de rabattement (9) orientée parallèlement à la ligne de pliage pivotante (8), et
- est aussi divisée en trois segments (7a, 7b, 7c) au moyen des lignes de pliage rabattables (10, 11) se prolongeant en outre en ligne droite dans la plaque latérale (7),
dans lequel la ligne de pliage pivotante (8) s'étend sur toute la longueur de la plaque de base (6) et de la plaque pivotante (5),
**caractérisé en ce que** le segment central (6b) des trois segments (6a, 6b, 6c) de la plaque de base (6) présente un motif de coupe (16) sous forme d'une pluralité de coupes longitudinales individuelles.

2. Emballage secondaire (1) selon la revendication 1, **caractérisé en ce que** la ligne de pliage de rabattement (9) s'étend sur toute la longueur de la plaque latérale (7) et de la plaque pivotante (5).

3. Procédé d'emballage secondaire d'un produit médical (3) en utilisant un emballage secondaire pliable (1) selon la revendication 1 ou 2 avec les étapes de procédé suivantes :
- pose d'un produit médical (3) sur le segment central (6b) des trois segments (6a, 6b, 6c) de la plaque de base (6) ;
- pivotement de la plaque pivotante (5) le long de la ligne de pliage pivotante (8) jusqu'à ce que la plaque pivotante (5) repose sur la plaque de base (6) en faisant passer le produit médical (3) à travers la découpe en forme de fenêtre (14) ;
- rabattement de la plaque latérale (7) le long de la ligne de pliage de rabattement (9) pour prendre en sandwich la plaque de base (6) entre la plaque latérale (7) et la plaque pivotante (5), et
- basculement des deux segments extérieurs respectifs (5a, 5c, 6a, 6c, 7a, 7c) des trois segments de la plaque de base (6), de la plaque pivotante (5) et de la plaque latérale (7) le long des lignes de pliage rabattables respectives (10, 11) en direction du produit médical (3).

4. Procédé selon la revendication 3, **caractérisé en ce que** le produit médical (3) est emballé dans un emballage secondaire stérile (1) avant d'être posé sur le segment central (6b) des trois segments (6a, 6b, 6c) de la plaque de base (6).

5. Procédé selon la revendication 3 ou 4, **caractérisé en ce que** l'emballage secondaire (1) est emballé avec le produit médical (3) dans une boîte d'emballage secondaire (15), de préférence normalisée, après le basculement des deux segments extérieurs respectifs (5a, 5c, 6a, 6c, 7a, 7c) des trois segments de la plaque de base (6), de la plaque pivotante (5) et de la plaque latérale (7) le long des lignes de pliage rabattables respectives (10, 11) en direction du produit médical (3).
